Europäisches Patentamt

European Patent Office

Office européen des brevets

(11) Publication number: **0 208 344 B1**

## EUROPEAN PATENT SPECIFICATION

(45) Date of publication of patent specification: **08.07.92** (51) Int. Cl.⁵: **G01N 33/20**

(21) Application number: **86200650.9**

(22) Date of filing: **16.04.86**

(54) **Limpet test cell.**

(30) Priority: **14.05.85 NL 8501398**

(43) Date of publication of application:
**14.01.87 Bulletin 87/03**

(45) Publication of the grant of the patent:
**08.07.92 Bulletin 92/28**

(84) Designated Contracting States:
**DE FR GB IT NL SE**

(56) References cited:
**FR-A- 2 349 833**
**GB-A- 564 516**
**GB-A- 2 092 304**
**US-A- 4 221 651**

**PATENT ABSTRACTS OF JAPAN, vol. 8, no.
105 (P-274)[1542], 17th May 1984; & JP-A-59
15 851 (TOKYO SHIBAURA DENKI K.K.)
26-01-1984**

**PATENT ABSTRACTS OF JAPAN, vol. 8, no.
48 (P-258)[1485], 3rd March 1984; & JP-A-58
198 757 (KAWASAKI SEITETSU K.K.)
18-11-1983**

(73) Proprietor: **SHELL INTERNATIONALE RE-
SEARCH MAATSCHAPPIJ B.V.
Carel van Bylandtlaan 30
NL-2596 HR Den Haag(NL)**

(72) Inventor: **DeLargy, Katrina Mary
P.O. Box 1
Chester Cheshire(GB)**
Inventor: **Harrison, Derek Roberts
P.O. Box 1
Chester Cheshire(GB)**

Rank Xerox (UK) Business Services

## Description

The invention relates to a limpet test cell for the in-situ testing of parts of steel surfaces.

With steel surfaces such as for example the hulls of ships, and the like, it is important to detect, identify and measure in situ the surface contamination. Moreover, it is important to be able to evaluate the condition of the coating of the steel surface.

Although several laboratory techniques are known for this purpose, they cannot be used in situ, for example under hard conditions such as in a shipyard during the construction or repair of ships (during dry-docking of ships) or in the course of offshore operations. The requirements that a test cell used in a shipyard and in offshore operations must satisfy are as follows:

1) The tests must be of brief duration.
2) The cell must be portable and simple to operate.
3) It must be possible to apply the test procedure on both horizontal and vertical surfaces and testing must be possible under relatively primitive conditions.
4) Operation must be intrinsically safe.

The invention provides a test cell that allows specific measurements to be made in a defined region of the surface to be tested and is suitable for in-situ semi-quantitative analysis of steel surfaces, for example those in tankers and during offshore operations. However, it will be appreciated that the use of the cell of the invention is not restricted to shipyards or offshore operations. The invention, therefore, provides a test cell for the in-situ testing of parts of steel surfaces, comprising: a cell body provided with a testing space that is, during use, directed towards the surface to be tested; means for the attachment of the cell body to the surface to be tested; means for the supply and removal of test liquid to and, respectively, from the testing space of the cell body after the cell has been attached to the surface to be tested, characterized in that the dimensions of the surface area of the testing space are several times greater than the depth of the testing space and that means are present for the detachment of the cell body from the surface to be tested after completion of the test, and said means for supply and removal of test liquid consist of holes in the cell body which are angled but not normal to the surface to be tested.

It is remarked that JP-A-5915851 discloses a test cell comprising two tanks: one of which contains electrolyte and the other contains an intermediate liquid with the two chambers being separated by a diaphragm. However, this known device is not suitable to be used in vertical position against a vertical wall surface. Further, this known design utilizes a deep and narrow tank, i.e. a relatively large volume of liquid samples a small surface only which is not economically.

According to the present invention a very shallow and relatively broad test volume is utilized so that a minimum volume of liquid samples a chosen area of the surface to be investigated, thereby maximizing from an economical viewpoint the concentration of surface salts and increasing the sensitivity of the process.

The invention will now be explained in further detail by way of example with reference to the accompanying drawings in which

fig.1 represents the side of the test cell according to the invention, which side is, during use, directed towards the steel surface to be tested such as for example the wall of a tank, watertight bulkhead or ship's hull; and

fig. 2 represents a cross-section of a test cell, showing further details of the cell of the invention.

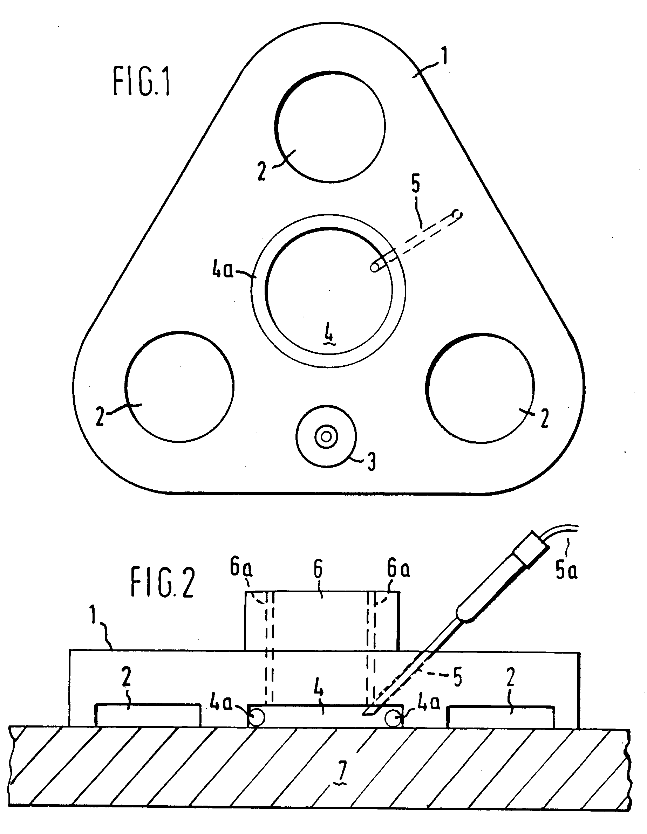

WIth reference to fig. 1 the test cell according to the invention comprises a testing body 1 which, in an advantageous embodiment, is triangular in shape and constructed of perspex.

The testing body 1 is provided with a plurality of means 2 for attachment of the cell body 1 to the surface to be tested. In fig. 1, only three of such means are shown, but any number suitable for the purpose can be used. In an advantageous embodiment these means are magnets.

Moreover, there is a means 3 fitted in order to enable the cell body to be detached from the surface to be tested after completion of the test. In an advantageous embodiment this means is a release screw.

The testing body 1 is provided on the side directed towards the surface to be tested with a testing space 4 which in an advantageous embodiment is of circular shape and provided with a sealing O-ring 4a to give a good seal between the testing space and the part of the metal surface that is not tested.

In this way, a limited region can be tested each time by relocating the cell. The testing space 4 is provided with means 5 for the supply (inlet ports) and removal of test liquids to and, respectively, from the testing space. An additional venting screw (not shown) for release of air during filling of the testing space may be present. The means 5 may, for example, be connected to a nozzle or probe (not shown in fig. 1). For example, solutions for the washing of the surface to be tested or electrolytes can be supplied and removed in order to perform certain specific tests. These specific tests will not be described in detail as they are not part of the invention.

For example, electrical conductimetric or potentiometric measurements can be made by fitting electrodes and metres to a connection block on the test cell (not shown in fig. 1).

It is also possible to supply and remove salt-free distilled water through the means 5 in order to rinse the surface to be tested and subsequently to supply reagents which change colour, for example, in the presence of certain conditions on the surface.

Potentiometric tests employ the potential difference between Fe and Zn in an electrolyte in order to determine the metallic nature of the surface to be tested.

Referring now to fig. 2 the cell body 1 is represented provided with a terminal block 6, which comprises any suitable electrodes 6a, 6b (schematically shown in dotted lines). The electrodes can for example be platinum electrodes. Metres may be incorporated or attached for specific tests. The metres and the electrical connections of the electrodes have not been shown for reasons of clarity.

The cell body 1 is further provided with suitable means 2 for magnet clamping to the metal surface 7 to be tested (for example a tank bottom, bulkhead, external hull and the like).

Fig. 2 also shows the testing space 4 and the sealing O-ring 4a. A release screw has not been represented for reasons of clarity. The testing space 4 is provided with an entry port 5. Through this entry port 5 a probe or syringe 5a may be inserted in order to be able to supply or remove test liquids or washing solutions and the like to and from the testing space 4.

The probe or syringe 5a may be connected by any suitable means to any further equipment suitable for the purpose (not shown for reasons of clarity).

It will be appreciated that any suitable means for supply or removal of such liquids or solutions to the testing space can be applied. For example, a nozzle can be used.

In particular, the cell of the invention can be used for measuring residual salt contamination following shot/compressed-air cleaning of rusted steel. Moreover, the cell of the invention can be used for detecting residual metallic pigments (Zn, Al) in paint coatings and also for assessing the cleanliness of the wall of a tank or ship during painting work.

It will be appreciated that the cell of the invention can be used in many other applications, for example on any structural steel such as chemical plants, bridges and so on during inspection/maintenance.

Further, the cell of the invention can also be used in steel inspection by ships personnel and/or paint manufacturers. For example, the cell of the invention can be used to examine sprayed or plated metal coatings.

In an advantageous embodiment of the invention the design of the cell may be modified to include means for scouring the surface or agitating the liquid to improve efficiency of washing/sampling, and inbuilt metres for more convenient operation/greater flexi-bility.

In a practical, non-limiting embodiment of the invention, the weight of the cell is 1,4 kilograms, the diameter of the testing surface is 5 cm, the testing volume is 10 ml, the side length of the (triangular) cell is 150 mm and the overall depth of the cell is 25 mm.

## Claims

1. A test cell for the in-situ testing of parts of steel surfaces, comprising: a cell body (1) provided with a testing space (4) that is, during use, directed towards the surface (7) to be tested; means (2) for the attachment of the cell body (1) to the surface (7) to be tested; means (5) for the supply and removal of test liquid to and, respectively, from the testing space (4) of the cell body (1) after the cell has been attached to the surface (7) to be tested, characterized in that the dimensions of the surface area of the testing space (4) are several times greater than the depth of the testing space (4) and that means (3) are present for the detachment of the cell body (1) from the surface (7) to be tested after completion of the test, and said means (5) for supply and removal of test liquid consist of holes in the cell body which are angled but not normal to the surface (7) to be tested.

2. The test cell as claimed in claim 1, characterized in that the means (2) for the attachment of the cell body (1) to the surface (7) to be tested consist of magnets.

3. The test cell as claimed in claims 1 or 2, characterized in that the detaching means (3) is a release screw.

4. The test cell as claimed in any one of claims 1-3, characterized in that the cell body (1) has a triangular shape.

5. The test cell as claimed in any one of claims 1-4, characterized in that the testing space (4) is provided with a sealing O-ring (4a).

6. The test cell as claimed in any one of claims 1-5, characterized in that the testing space (4) is circular.

7. The test cell as claimed in any one of claims 1-5, characterized in that means (6a) for making potential/conductivity measurements are present.

8. The test cell as claimed in claim 7, characterized in that a terminal block (6) comprising electrodes (6a) is present.

**Revendications**

1. Une cellule de test pour tester in-situ des pièces ayant des surfaces en acier, comprenant: un corps de cellule (1) muni d'un espace de test (4) qui, pendant l'utilisation, est dirigé vers la surface (7) devant être testée ; des moyens (2) pour fixer le corps de la cellule (1) à la surface (7) devant être testée ; des moyens (5) pour la fourniture et le retrait de liquide de test respectivement vers et à partir de l'espace de test (4) du corps de la cellule (1) après que la cellule ait été fixée à la surface (7) devant être testée, caractérisée en ce que les dimensions de la surface de l'espace de test (4) sont beaucoup plus grandes que la profondeur de l'espace de test(4), en ce que des moyens(3)sont présents pour détacher le corps de la cellule (1)de la surface (7) devant être testée après que le test ait été effectué, et en ce que lesdits moyens (5) pour la fourniture et le retrait de liquide de test sont composés d'ouvertures dans le corps de la cellule qui forment un angle mais ne sont pas normaux par rapport à la surface (7) devant être testée.

2. La cellule de test selon la revendication 1, caractérisée en ce que les moyens (2) pour détacher le corps de la cellule (1) de la surface (7) devant être testée sont composés d'aimants.

3. La cellule de test selon la revendication 1 ou 2, caractérisée en ce que les moyens pour détacher (3) sont une vis de déblocage.

4. La cellule de test selon l'une des revendications 1 à 3, caractérisée en ce que le corps de la cellule (1) a une forme triangulaire.

5. La cellule de test selon l'une des revendications 1 à 4, caractérisée en ce que l'espace de test (4) est muni d'un joint torique d'étanchéité (4a).

6. La cellule de test selon l'une des revendications 2 à 5, caractérisée en ce que l'espace de test(4) est circulaire.

7. La cellule de test selon l'une des revendications 1 à 5, caractérisée en ce que des moyens (6a) servant à effectuer les mesures de potentiel/conductivité sont présents.

8. La cellule de test selon la revendication 7, caractérisée en ce qu'un bornier (6) comprenant des électrodes (6a) est présent.

**Patentansprüche**

1. Prüfzelle zum in-situ Prüfen von Teilen stählerner Oberflächen mit: einem Zellenkörper (1), der einen Prüfraum (4) aufweist, welcher im Gebrauch zu der zu prüfenden Oberfläche (7) weist, Mitteln (2) zur Befestigung des Zellenkörpers (1) auf der zu prüfenden Oberfläche (7), Mitteln (5) für die Zufuhr und Entnahme von Prüfflüssigkeit in den und aus dem Prüfraum (4) des Zellenkörpers (1) nachdem die Zelle an der zu prüfenden Oberfläche (7) angebracht ist,
dadurch **gekennzeichnet,** daß die Abmessungen der Oberfläche des Prüfraums (4) mehrere Male größer sind als die Tiefe des Prüfraums (4), und daß Mittel (3) zum Lösen des Zellenkörpers (1) von der zu prüfenden Oberfläche (7) nach Beendigung des Prüfvorgangs vorhanden sind, und daß die Mittel (5) zur Zufuhr und zur Entnahme von Prüfflüssigkeit aus Löchern im Zellenkörper bestehen, die in einem Winkel, jedoch nicht normal zu der zu prüfenden Oberfläche (7) angeordnet sind.

2. Prüfzelle nach Anspruch 1,
dadurch **gekennzeichnet,** daß die Mittel (2) zur Anbringung des Zellenkörpers (1) an der zu prüfenden Oberfläche (7) aus Magneten bestehen.

3. Prüfzelle nach Anspruch 1 oder 2,
dadurch **gekennzeichnet,** daß die Mittel (3) zum Lösen aus einer Löseschraube bestehen.

4. Prüfzelle nach einem der Ansprüche 1 bis 3,
dadurch **gekennzeichnet,** daß der Zellenkörper (1) eine dreieckige Gestalt aufweist.

5. Prüfzelle nach einem der Ansprüche 1 bis 4,
dadurch **gekennzeichnet,** daß der Prüfraum (4) einen dichtenden O-Ring (4a) aufweist.

6. Prüfzelle nach einem der Ansprüche 1 bis 5,
dadurch **gekennzeichnet,** daß der Prüfraum (4) kreisförmig ist.

7. Prüfzelle nach einem der Ansprüche 1 bis 5,
dadurch **gekennzeichnet,** daß Mittel (6a) zur Durchführung zur Potential-/Leitfähigkeitsmessungen vorhanden sind.

8. Prüfzelle nach Anspruch 7,
dadurch **gekennzeichnet,** daß eine Anschlußleiste (6) mit Elektroden (6a) vorhanden ist.

FIG.1

FIG.2